# EUROPEAN PATENT APPLICATION

(11) **EP 1 938 815 A1**
(43) Date of publication of application: **02.07.2008**
(21) Application number: 06810487.6
(22) Date of filing: 25.09.2006
(51) Int. Cl.: A61K 31/203, A61K 31/196, A61P 27/02

(54) **PHARMACEUTICAL FOR PREVENTION AND TREATMENT OF OPHTHALMIC DISEASE INDUCED BY INCREASE IN VASOPERMEABILITY**

(30) Priority: 27.09.2005 JP 2005280166
(71) Applicant: Sapporo Medical University, Hokkaido 060-8556 (JP)
(72) Inventor: SAWADA, Norimasa, Sapporo-shi, Hokkaido 060-8556 (JP); OSANAI, Makoto, Sapporo-shi, Hokkaido 060-8556 (JP); NISHIKIORI, Nami, Sapporo-shi, Hokkaido 060-8556 (JP)
(74) Representative: Polypatent
(86) International application number: PCT/JP2006/318919
(87) International publication number: WO 2007/037188

(57) **Abstract**

A medicament for preventive and/or therapeutic treatment of an ocular disease resulting from vascular hyperpermeability, for example diabetic retinopathy or age-related macular degeneration, which comprises as an active ingredient a retinoid such as all trans retinoic acid or 4-[(5,6,7,8-tetrahydro-5,5,8,8-tetramethyl-2-naphthalenyl)carbamoyl]benzoic acid.

## Description

### Technical Field

The present invention relates to a medicament for preventive and therapeutic treatment of ocular diseases. More specifically, the invention relates to a medicament useful for preventive and/or therapeutic treatment of ocular diseases resulting from vascular hyperpermeability such as diabetic retinopathy and age-related macular degeneration which comprises a retinoid such as RARα agonist as an active ingredient.

### Background Art

In recent years, the number of patients with diabetes in Japan is reported to be more than six million, and the number of the patient has been constantly increasing. Diabetic retinopathy, one of the three major complications of diabetes, is the primary cause of the acquired blindness. It is considered that four thousand people become blind every year due to this cause, which has become a serious social problem as well as a problem in medical and ophthalmologic field. Diabetic retinopathy is a retinal blood vessel lesion with advances of pathological conditions from vascular hyperpermeability of retinal capillaries, through obstruction of capillaries to angiogenesis, which finally leads to loss of eyesight. Diabetic retinopathy develops gradually after diabetes onset, and becomes symptomatic 10 to 20 years after the diabetes onset.

In the western countries, loss of eyesight resulting from age-related macular degeneration (AMD) has been in the first ratio. This disease has been very rapidly increasing recently also in Japan and has become a social problem. AMD is a disease caused on the yellow spot of the eyes of the aged people, and develops based on the aging change of retina pigment epithelium cells, Bruch film, and the choroid membrane blood vessel. It is not rare that AMD onset on both eyes, and in particular, AMD classified as the exudative type may readily lead to loss of eyesight for which no therapeutic treatment is effective. Therefore, the disease has become a serious clinical problem. A major symptom of AMD is a choroidal neovascular produced on the yellow spot. However, no effective therapeutic treatment is available for the disease, since in many clinical cases a conventional therapy such as laser photocoagulation is not applicable due to peculiarity that the lesion generates on the yellow spot.

Pathological neovascularization in the retina and choroid membrane is significantly involved in these pathological conditions of the eye-ground-disease which are major causes for the loss of eyesight. It is considered, however, that an increase in permeability of the retina capillary occurs as an early change prior thereto. If it is possible to prevent the increase of vascular permeability, it is considered that preventive and/or therapeutic treatment is achievable for advances of pathological conditions of diabetic retinopathy, age-related macular degeneration and the like.

Epithelial cells of multicellular organism develop a particular type of intercellular adhesion structure and constitute a closed cavity structure that has an independent inner environment. For example, the cardiovascular system is a closed cavity system that is surrounded by vascular endothelial cells, and in the gastrointestinal tract, the mucosal epithelial cells separate the outer and inner part of the body. At the same time, epithelial cells form a barrier to separate the inner environment from the outer environment. These barriers are called Blood-tissue barriers (BTB) and have a function to separate specific organs such as central nervous system, retina and testis from the blood. The body of such a biological barrier consists of tight junction that seals the cell-cell interspaces (paracellular pathway).

Since the tight junction plays an important role as a biological barrier in order to maintain the homeostasis of the individual, dysfunction of the tight junction is deeply involved in the pathological conditions of disorders such as edema, jaundice, diarrhea and the like (for example, see, non-patent document 1 to 3). However, the regulatory mechanism of the tight junction has not been fully elucidated, and only limited numbers of reports of analysis using human tissue are available. Therefore, elucidation of the regulatory mechanism of the tight junction and study of organ-specific regulatory mechanism thereof will become an important knowledge that directly relates to the regulation of the onset of the diseases.

In many ocular diseases including diabetic retinopathy and age-related macular degeneration, function of the tight junction of the blood retinal barrier (BRB) is decreased and vascular permeability is often increased from an early stage of pathological conditions. As retinal blood circulation, there are retinal circulation and choroid circulation. A barrier exists in each of the circulations of which essential structure is a tight junction of the vascular endothelial cells. The BRB is formed by both of the retinal circulation and choroid circulation to maintain the homeostasis of the retina. The microvascular endothelial cells form inner BRB (i-BRB) in the retinal circulation. Especially i-BRB plays an important role for maintaining the homeostasis of the retina. Accordingly, its failure will cause variety of ocular pathological conditions including retinal edema, which may result in a decrease or loss of eyesight of patients with diabetic retinopathy and age-related macular degeneration.

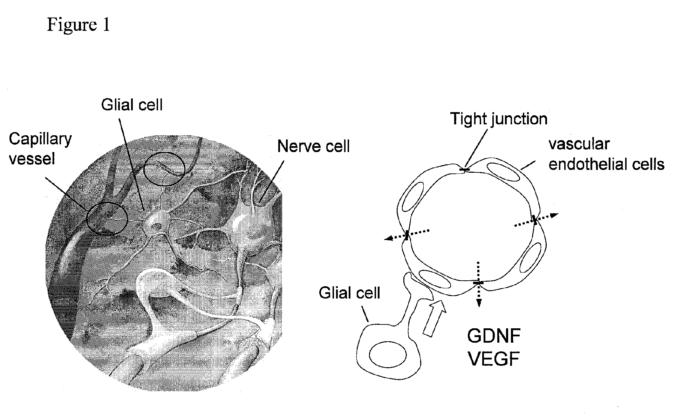

In diabetes, many functional abnormalities are caused by abnormality in the cytokine expression with hyperglycemic condition, abnormal metabolic processes, and abnormality of dynamics of blood flow, all of them are complicatedly correlated. A cause that ultimately increases permeability of retinal capillaries is vascular endothelial growth factor (VEGF) produced at retina. VEGF is also called as vascular permeability factor (VPF) which enhances neovascularization and also act as a potent vascular hyperpermeability factor. The i-BRB has a characteristic histological structure in which a dense cell sheet is formed with capillary vascular endothelial cells on the basis of the presence of continuous tight junction between capillary vessel cells, and the sheet is surrounded by glial cells from the outside (Figure 1). Therefore, in this biological unit, existence of mutual functional relationship is presumed therebetween consisting of the glial cells and vascular endothelial cells. The expression of VEGF is in fact widely known to be induced by hyperglycemic condition, abnormal sugar metabolism, abnormality in cytokine expression, ischemia, or hypoxic condition. VEGF-producing cells in the retina are mainly considered to be retinal glial cells.

However, an increase in the VEGF expression and vascular hyperpermeability resulting therefrom occur prior to clinically apparent lumen occlusion of retinal blood vessels. Therefore, if it is possible to modify characters of glial cells to maintain their functions at normal level, a method for therapeutic treatment can be established against the vascular hyperpermeability caused by the decrease in the barrier function of the vascular endothelial cells, as early stage pathologic changes in diabetic retinopathy and age-related macular degeneration, by recovering the function of the tight junction to the normal condition, thereby an effective method for therapeutic treatment for these diseases can be provided.

The inventors of the present invention have so far focused on the phenomenon of the vascular hyperpermeability in the ocular diseases such as diabetic retinopathy, and conducted researches by using porcine cerebral cortical microvascular endothelial cells that constitute the blood brain barrier (BBB) which form a quite similar biological unit to BRB, and also the human brain astrocyte which is classified into glial cell, in order to clarify the functional regulation of the tight junction of the vascular endothelial cells. The inventors first established the separation method and cultural procedures of the porcine brain vascular endothelial cells, and showed that the tight junction function of the vascular endothelial cells that form the BBB is controlled by cAMP (non-patent document 4). When cAMP is acted on the porcine brain vascular endothelial cells, transepithelial electrical resistance (TER) which is the index of tight junction barrier function (TER becomes higher at higher barrier function) increased 3 to 4-fold. They also found that the glial cell line-derived neurotrophic factor (GDNF), having a life-supporting effect for nerve cells that was secreted from the glial cells, increased the barrier function of the tight junction of the brain vascular endothelial cells that formed the BBB (non-patent document 5). They further showed that GDNF receptor (GFR α 1) became expressed in the capillary vessel of the brain cortex with the maturing of BBB (non-patent document 6), and that GDNF was also secreted from the retinal glial cells (non-patent document 7). In addition, they revealed that the GDNF had an effect of enhancing the barrier function of the retinal blood vessel.

The inventors further focused on AGE (advanced end glycation product) as a mechanism to cause hyperpermeability of the retinal blood vessel that was an early stage development of the ocular diseases such as diabetic retinopathy, and conducted researches under mechanism assumption that VEGF secretion at the retina glial cells is increased with AGE, whilst the expression of the endogenous GDNF is decreased (non-patent document 8). AGE, a final product of late glycosylation synthesized by a non-enzymatic reaction due to chronic hyperglycemia, is considered not to be a single substance but to be consisted of variety of substances on the basis of the nature of the reaction. It is reported that at least five kinds of AGEs, carboxymethyl-lysine (CML), and carboxyethyl-lysine (CEL) were present in serum of a diabetic patient. When the porcine brain vascular endothelial cells were subcultured and acted with various kinds ofAGEs to measure the TER as an index of the barrier function of the vascular endothelial cells, no significant difference was observed in the TER by treatment with any kind of AGE after 24 hours compared to the control.

Whilst when the human brain astrocyte was treated in the same manner, the AGE (AGE-2) that was added with a glyceraldehyde gave the highest decrease in the expression of GDNF, as well as the highest increase of the expression of VEGF. AGE-2 enhances the expression of VEGF of the glial cells in vitro. It is reported that when AGE-2 was injected in the rat vitreum or intraperitoneally, the expression of VEGF was also enhanced in vivo mainly around retina glial cells. When AGE-2 and VEGF were searched immunohistochemically in human diabetic retina, the deposition of AGE-2 was observed at the wall of the retinal blood vessel or the glial cells around retina, and its distribution is reported to be similar to that of the expression of VEGF protein, which suggests that the target cells of AGE-2 are glial cells.

From the studies mentioned above, it was revealed that the microvascular hyperpermeability due to the reduced function of the tight junction of the BRB in the pathologic condition of diabetic retinopathy and the like is brought as a result of a change of expression of GDNF and VEGF that regulate the vascular permeability by the action of mainly AGE-2, among the several ACEs that are synthesized non-enzymatically under the condition of the chronic hyperglycemic state, against glial cells as target cells inside the biological functional unit called as BBB formed by the vascular endothelial cells and the glial cell (non-patent document 8).

The inventors of the present invention have shown that all-trans retinoic acid (atRA), which is one of the retinoic acid (RA) that is a ligand of nuclear receptor (non-patent document 9), is essential in the formation process of the tight junction through the dimer of retinoid X receptor (RXR))/retinoic acid receptor (RAR) which is an RA specific nuclear receptors, and forms epithelial cell polarity through the control function of transcription level (non-patent document 10). However, the non-patent document 10 relates to studies on cells different from retinal cells, and to studies of the expression of genes that code the tight junction and the barrier in F9 cells in the endoderm differentiation. Non-patent document 10 shows that atRA directly acted on the epithelial cell. However, as will be explained below, the medicament of the present invention has the mode of action to be involved through paracrinic cooperation of epithelial cells and glial cells, and therefore, their modes of action are totally different to each other.

The inventors of the present invention also showed that the barrier function of the vessel was increased when the lung-derived vascular endothelial cells was treated with atRA (non-patent document 11). By the administration of RA to a tumor tissue, a decrease in VEGF secreted from the tumor cells occurred, and as a result, the inhibition of tumor neovascularization was observed, whilst neovascularization in enbriotic life is inhibited by the interception of RA signal. This result suggests that the RA signal at the transcription level plays an important role in angiogenesis and the vascular function. Therefore, it is presumed that the signal-transducing pathway through RA effects on the vascular endothelial cells, and has some kind of functional relation with their tight junction function.

However, no studies have been made so far as to whether a change in a gene expression of the astrocyte by a specific substance acts in a paracrinic manner to change the permeability of vascular endothelial cells in a co-culture system of astrocyte and vascular endothelial cells, and further, with diabetic model mice chemically induced by streptozotocin, as to whether such specific substance significantly suppresses the increase of vascular permeability in the hyperglycemic condition in a group of animals with early stage diabetes and that of animals with middle stage diabetes, and a s result, is effective as a medicament for preventive and therapeutic treatment of diabetic retinopathy and the like.
[Non-patent document 1] "Kekkan zoki kannmon to shikkan", Mori, Michio author and editor, "Byouki to Saibounaishoukikan", Bunkoudou, pp.182-194, 2002
[Non-patent document 2] "Sukima" no seibutsugaku -Saibou kan secchaku souchi taito ketsugou to hito shikkan-", Sapporo Igaku Zasshi, 72, pp.1-7, 2003
[Non-patent document 3] Med. Electron Microsc., 36, pp.147-156, 2003
[Non-patent document 4] Exp. Cell Res., 290, pp.275-288, 2003
[Non-patent document 5] Biochem. Biophys. Res. Commun., 261, pp.108-112, 1999
[Non-patent document 6] Am. J. Physiol. Cell Physiol., 279, C361-368, 2000
[Non-patent document 7] Cell Struct. Funct., 25, 237-241, 2000
[Non-patent document 8] Biochem. Biophys. Res. Commun., 330, 361-366, 2005
[Non-patent document 9] FASEB J., 10, pp.940-954, 1996
[Non-patent document 10] Exp. Cell Res., 263, pp.163-172, 2001.
[Non-patent document 11] Exp. Cell Res., 222, pp.269-274, 1996.

### [Disclosure of Invention]

### [Object to b achieved by the invention]

An object of the present invention is to provide a medicament for preventive and/or therapeutic treatment of ocular diseases resulting from vascular hyperpermeability, for example, ocular diseases such as diabetic retinopathy and age-related macular degeneration.

### [Means to solve the object]

The inventor of the present invention found that when retinoids, such as RARα agonist that act on the RARα receptor, were administered to diabetic model mice chemically induced by streptozotocin, the enhancement of the vascular permeability at the hyperglycemic condition was significantly suppressed in the group of early stage diabetes and in the group of middle stage diabetes as compared to the non-administration group. The inventors also found that the retinoids had high effectiveness as an active ingredient of a medicament for preventive and/or therapeutic treatment of ocular diseases resulting from vascular hyperpermeability, for example ocular diseases such as diabetic retinopathy and age-related macular degeneration. The present invention was achieved on the basis of the above findings.

The present invention thus provides a medicament for preventive and/or therapeutic treatment of an ocular disease resulting from vascular hyperpermeability, which comprises a retinoid as an active ingredient.
According to a preferred embodiment of the above invention, there is provided the aforementioned medicament wherein the ocular disease resulting from vascular hyperpermeability is diabetic retinopathy or age-related macular degeneration.
There are also provided the aforementioned medicament used for prevention of diabetic retinopathy at early stage diabetes or middle stage diabetes; and the aforementioned medicament used for prevention at presymptomatic stage of diabetic retinopathy.
According to further preferred embodiments of the above invention, provided are the aforementioned medicament, wherein the retinoid is all-trans retinoic acid; the aforementioned medicament, wherein the retinoid is non-natural retinoid; and the aforementioned medicament, wherein the retinoid has a basic skeleton comprising an aromatic ring bound with an aromatic carboxylic acid or tropolone by means of a bridging group.

According to still further preferred embodiments of the above invention, provided are the aforementioned medicament, wherein the retinoid is capable of binding to retinoic acid receptor (RAR) subtype α and subtype β; the aforementioned medicament, wherein the retinoid is capable of binding to retinoid X receptor X (RXR); the aforementioned medicament, wherein the retinoid has a basic skeleton comprising a substituted phenyl group bound with benzoic acid or tropolone by means of a bridging group; the aforementioned medicament, wherein the retinoid is Am80 (4-[(5,6,7,8-tetrahydro-5,5,8,8-tetramethyl-2-naphthalenyl)carbamoyl]benzoic acid) or Am580 (4-[(5,6,7,8-tetrahydro-5,5,8,8-tetramethyl-2-naphthalenyl)carboxyamide]-benzoic acid); the aforementioned medicament, wherein the retinoid comprises dibenzo[b,f][1,4]thiazepinylbenzoic acid as a basic skeleton; the aforementioned medicament, wherein the retinoid is 4-[2,3-(2,5-dimethyl-2,5-hexano)dibenzo-[b,f][1,4]-thiazepin-11-yl]benzoic acid; and the aforementioned medicament, wherein the retinoid is 4-[5-(4,7-dimethylbenzofuran-2-yl)pyrrol-2-yl]benzoic acid.

From another aspect, provided are use of the above retinoid for manufacture of the aforementioned medicament; and a method for preventive and/or therapeutic treatment of an ocular disease resulting from vascular hyperpermeability, preferably diabetic retinopathy or age-related macular degeneration, comprising the step of administering an effective amount of the above retinoid to a mammal including a human.

### [Brief Explanation of Drawings]

[Fig. 1] A schematic illustration showing the biological functional unit consisting of the vascular endothelial cells and the glial cells.
[Fig. 2] A figure showing the expression of GDNF by the atRA treatment in human glioma cells.
[Fig. 3] A figure showing the outline of the apparatus to measure the paracrinic effect using the co-culture system of the vascular endothelial cells and the astrocyte.
[Fig. 4] A figure explaining the modulatory effect of the barrier function of the vascular endothelial cells by all-trans retinoic acid (atRA).
[Fig. 5] A figure showing that the substances of the present invention had effectiveness on the barrier function as well as atRA.
[Fig. 6] A figure showing the increase in the GDNF expression by the substances of the present invention.
[Fig. 7] A figure showing that atRA and Am580 suppressed the decrease of GDNF expression by AGE-2.
[Fig. 8] A figure showing that the astrocyte-derived GDNF modulated by atRA changed the barrier function of the vascular endothelial cells.
[Fig. 9] A figure showing the effects of the substances of the present invention to the diabetic model mice.

### [Best Mode for Carrying out the Invention]

In the specification, the term "retinoid" means compounds that bind to receptors required for all trans-retinoic acid (atRA) and 9-cis-retinoic acid to exhibit physiological functions thereof, and thereby exhibit actions similar to those of retinoic acid or a part of the actions, and the term means compounds that have at least one retinoid-like action, for example, one ore more of cell differentiating action, cell proliferation promoting action, life supporting action, and the like. Whether a certain compound is a retinoid or not can be readily determined by the method described in H. de The, A. Dejean, "Retinoids: 10 years on.", Basel, Karger, 1991, pp.2-9.

Further, while retinoids generally have a property of binding to a retinoic acid receptor (RAR), and sometimes have property of binding to RXR together with RAR, the retinoid used as the active ingredient of the medicament of the present invention is preferably a retinoid that binds to the subtype α of RAR to exhibit an agonistic action. Whether a certain compound is an agonist of RARα or not, also as for an agonist of a retinoic acid receptor subtype, can be readily determined by the method of the aforementioned publication.

As the active ingredient of the medicament of the present invention, any of natural retinoids or non-natural retinoids may be used. Preferably, all trans retinoic acid among natural retinoids, or non-natural retinoids may be used. As the non-natural retinoids, those having a basic skeleton comprising an aromatic ring bound with an aromatic carboxylic acid or tropolone by means of a bridging group may be used.

More specifically, as non-natural retinoids, those represented by the following general formula: B-X-A (wherein B represents an aromatic group which may be substituted, X represents a bridging group, and A represents a carboxylic acid-substituted aromatic group or tropolonyl group) can be used.

As the aromatic group represented by B, a phenyl group which may have a substituent is preferred. Type, number, and substituting position of the substituent on the phenyl group are not particularly limited. As the substituent on the phenyl group, for example, a lower alkyl group can be used (in the specification, the term "lower" means a carbon number of 1 to about 6, preferably 1 to 4). As the lower alkyl group, an alkyl group having a linear or branched chain is preferred, and more specific examples include methyl group, ethyl group, n-propyl group, isopropyl group, n-butyl group, sec-butyl group, tert-butyl group, and the like. Other examples of the substituent on the phenyl group include, for example, a lower alkoxyl group such as methoxy group, a halogen atom (the halogen atom may be any of fluorine atom, chlorine atom, bromine atom, and iodine atom), a lower alkyl-substituted silyl group such as trimethylsilyl group, and the like. As the phenyl group, for example, a phenyl group substituted with 2 to 4 of lower alkyl groups, a phenyl group substituted with 1 or 2 of tri(lower alkyl)silyl group, and the like are preferred, and a phenyl group substituted with 2 to 4 of alkyl groups, a phenyl group substituted with 2 of trimethylsilyl groups, and the like are more preferred.

When two of the lower alkyl groups substituting on the phenyl group are adjacent to each other, they may combine together to form one or two, preferably one of 5- or 6-membered ring together with the ring-constituting carbon atoms of the phenyl group to which they bind. The ring formed as described above may be saturated or unsaturated, and one or more lower alkyl groups such as methyl group and ethyl group may substitute on the ring. On the aforementioned formed ring, preferably 2 to 4 of methyl groups, more preferably 4 of methyl groups, may substitute. For example, it is preferred that two adjacent lower alkyl groups which substitute on the phenyl ring combine together to form 5,6,7,8-tetrahydronaphthalene ring, 5,5,8,8-tetramethyl-5,6,7,8-tetrahydronaphthalene ring, or the like. As the aromatic group represented by B, an aromatic heterocyclic group may also be used. Examples of such retinoid include a retinoid wherein B is a benzofuranyl group which may have a substituent, preferably benzofuran-2-yl group, particularly preferably 4,7-dimethylbenzofuran-2-yl group.

As the carboxylic acid-substituted aromatic group represented by A, a carboxylic acid-substituted phenyl group, a carboxylic acid-substituted heterocyclic group, and the like can be used, and 4-carboxyphenyl group is preferred. Examples of the heterocyclic carboxylic acid constituting the carboxylic acid-substituted heterocyclic group represented by A include, for example, pyrimidine-5-carboxylic acid, and the like. As the tropolonyl group represented by A, tropolon-5-yl group is preferred. On the ring of the carboxylic acid-substituted aromatic group or tropolonyl group, one or more substituents may exist.

Type of the bridging group represented by X is not particularly limited, and examples include, for example, -NHCO-, -CONH-, -N(R^{A})- (R^{A} represents a lower alkyl group, for example, cyclopropylmethyl group and the like), -C(R^{B})(R^{C})- (R^{B} and R^{C} independently represent hydrogen atom, a lower alkyl group, and the like). Further, X may be a divalent aromatic group. For example, X may be pyrrol-diyl group, or the like. Furthermore, the bridging group represented by X and the aromatic group represented by B may combine together to form a ring structure. For example, the basic skeleton of the retinoid represented by B-X-A may be dibenzo[b,f][1,4]thiazepinylbenzoic acid or dibenzo[b,f][1,4]diazepinylbenzoic acid. In the specification, the term "basic skeleton" means a main chemical structure for one or more arbitrary substituents to bind thereto.

As preferred retinoids, all-trans retinoic acid as natural retinoic acid and non-natural retinoid, for example, retinoids comprising a phenyl-substituted carbamoylbenzoic acid or a phenyl-substituted carboxamidobenzoic acid as a basic skeleton can be used. Various retinoids comprising a phenyl-substituted carbamoylbenzoic acid or a phenyl-substituted carboxamidobenzoic acid as a basic skeleton are known. Typical examples of retinoids having a phenyl-substituted carbamoylbenzoic acid as a basic skeleton include Am80 (4-[(5,6,7,8-tetrahydro-5,5,8,8-tetramethyl-2-naphthalenyl)carbamoyl]benzoic acid (refer to Hashimoto, Y., Cell Struct. Funct., 16, pp.113-123, 1991; Hashimoto, Y., et al., Biochem. Biophys. Res. Commun., 166, pp.1300-1307, 1990), and typical examples of retinoids having a phenyl-substituted carboxamidobenzoic acid include Tac101 (4-[(3,5-bis-trimethylsilylphenyl)carboxamido]benzoic acid (J. Med. Chem., 33, pp.1430-1437, 1990).

Preferred retinoids include, for example, compounds represented by the following general formula (I): 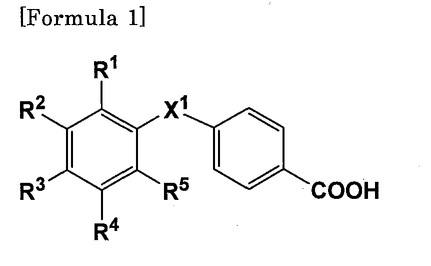 wherein R¹, R², R³, R⁴, and R⁵ independently represent hydrogen atom, a lower alkyl group, or a lower alkyl-substituted silyl group, when two of adjacent groups among R¹, R², R³, R⁴, and R⁵ are lower alkyl groups, they may combine together to form a 5- or 6-membered ring together with the carbon atoms of the benzene ring to which they bind (this ring may have one or more alkyl groups), and X¹ represents -CONH- or -NHCO-.

In the aforementioned general formula (I), as the lower alkyl group represented by R¹, R², R³, R⁴, and R⁵, a linear or branched alkyl group having 1 to about 6 carbon atoms, preferably 1 to 4 carbon atoms, can be used. For example, methyl group, ethyl group, n-propyl group, isopropyl group, n-butyl group, sec-butyl group, tert-butyl group, and the like can be used. On the aforementioned lower alkyl group, one or more arbitrary substituents may exist. Examples of the substituents include, for example, hydroxyl group, a lower alkoxyl group, a halogen atom, and the like. Examples of the lower alkyl-substituted silyl group represented by R¹, R², R³, R⁴, and R⁵ include, for example, trimethylsilyl group, and the like.

Two of adjacent lower alkyl groups selected from the group consisting of R¹, R², R³, R⁴, and R⁵ may combine together to form one or two, preferably one of 5- or 6-membered ring together with the carbon atoms of the benzene ring to which they bind. The ring formed as described above may be saturated or unsaturated, or an aromatic ring, and one or more lower alkyl groups such as methyl group and ethyl group may substitute on the ring. As the alkyl group which may substitute on the ring, a linear or branched alkyl group having 1 to about 6 carbon atoms, preferably 1 to 4 carbon atoms, can be used. For example, methyl group, ethyl group, and the like can be used, and preferably 2 to 4 of methyl groups, more preferably 4 of methyl groups, may substitute. For example, it is preferred that 5,6,7,8-tetrahydronaphthalene ring, 5,5,8,8-tetramethyl-5,6,7,8-tetrahydronaphthalene ring, or the like is formed by the benzene ring on which R² and R³ substitute, and R² and R³.

Examples of other preferred retinoids include, for example, retinoids comprising dibenzo[b,f][1,4]thiazepinylbenzoic acid or dibenzo[b,f][1,4]-diazepinylbenzoic acid as the basic skeleton represented by B-X-A. Examples of such retinoids are described in, for example, Japanese Patent Unexamined Publication (KOKAI) No. 10-59951. Particularly preferred examples of such retinoids include, for example, HX630 (4-[2,3-(2,5-dimethyl-2,5-hexano)dibenzo[b,f][1,4]-thiazepin-11-yl]benzoic acid). Further, examples of retinoids wherein X is -N(R^{A})-, and B is an aromatic heterocyclic carboxylic acid include, for example, 2-[2-(N-5,6,7,8-tetrahydro-5,5,8,8-tetramethyl-2-naphthalenyl-N-cyclopropylmethyl)a mino]pyrimidine-5-carboxylic acid. Further, examples of retinoids wherein X is a divalent aromatic group include, for example,
4-[5-(4,7-dimethylbenzofuran-2-yl)pyrrol-2-yl]benzoic acid. Examples of the compound wherein A is a tropolonyl group include, for example,
5-[[5,6,7,8-tetrahydro-5,5,8,8-tetramethyl-2-naphthalenyl]carboxamido]tropolone, and the like.
Examples of most preferred retinoids include Am80
(4-[(5,6,7,8-tetrahydro-5,5,8,8-tetramethyl-2-naphthalenyl)carbamoyl]benzoic acid) and Am580
(4-[(5,6,7,8-tetrahydro-5,5,8,8-tetramethyl-2-naphthalenyl)carboxamide]benzoic acid).

As the active ingredient of the medicament of the present invention, salts of the above retinoids may be used. For example, physiologically acceptable salts including metal salts such as sodium salts, potassium salts, magnesium salts, and calcium salts, ammonium salts, organic amine salts such as triethylamine salts, and ethanolamine salts, and the like can be used as the active ingredient of the medicament of the present invention. As the active ingredient of the medicament of the present invention, a prodrug of the above retinoid may be used. The term "prodrug" means a compound or a salt thereof which is, after oral or parenteral administration to a mammal, subjected to a structural change such as hydrolysis in vivo, preferably in blood, to produce the above retinoid or a salt thereof. For example, various means for producing prodrugs from pharmaceutical compounds having carboxylic acid, amino group, hydroxyl group or the like are known, and one of ordinary skill in the art can choose appropriate means. Types of the prodrug of the retinoid or a salt thereof are not particularly limited. For example, where a retinoid has carboxylic acid, an example includes a prodrug wherein the carboxylic acid is converted into an alkoxycarbonyl group. Preferred examples include ester compounds such as those formed with methoxycarbonyl group or ethoxycarbonyl group.

The aforementioned retinoid may have one or more asymmetric carbons depending on the types of substituents, and any optical isomers based on these asymmetric carbons, any mixtures of optical isomers, racemates, diastereoisomers based on two or more asymmetric carbons, any mixtures of diastereoisomers, and the like can be used as the active ingredient of the medicament of the present invention. Furthermore, geometrical isomers based on cis- or trans-configuration of double bond, any mixtures of geometrical isomers, and any hydrates or solvates of the compounds in free forms or in the form of a salt can also be used as the active ingredient of the medicament of the present invention.

The medicament of the present invention can be used for preventive and/or therapeutic treatment of ocular diseases resulting from vascular hyperpermeability. Typical examples of the ocular diseases resulting from vascular hyperpermeability include diabetic retinopathy and age-related macular degeneration. By suppressing the vascular hyperpermeability in these diseases, the medicament of the present invention can prevent the onset of said disease, and/or can achieve amelioration or remission of symptoms of said disease or inhibit advance of the symptoms.

The medicament of the present invention comprises, as an active ingredient, one or two or more substances selected from the group consisting of the aforementioned retinoid and a salt thereof, and a hydrate thereof and a solvate thereof. A preferred effectiveness may sometimes be obtained by administration of two or more different retinoids in combination. As the medicament of the present invention, the aforementioned substance, per se, may be administered. Preferably, the medicament can be administered as a pharmaceutical composition for oral or parenteral administration which can be prepared by a method well known to one of ordinary skill in the art.

As pharmaceutical compositions suitable for oral administration, examples include tablets, capsules, subtilized granules, granules, powders, liquids, and syrups. As pharmaceutical compositions suitable for parenteral administration, examples include injections, suppositories, inhalant, eye drops, nasal drops, ointments, creams, ophthalmic ointment, and plasters. Two or more pharmaceutical compositions may be used in combination. Preferred forms of the medicament of the present invention include pharmaceutical compositions for oral administration, as well as pharmaceutical compositions for parenteral administration such as eye drops or ophthalmic injections.

The aforementioned pharmaceutical composition can be prepared by adding one or two or more kinds of pharmacologically and pharmaceutically acceptable additives for pharmaceutical preparations. Examples of the additives for pharmaceutical preparations include, for example, excipients, disintegrators or disintegrating aids, binders, lubricants, coating agents, dyes, diluents, bases, dissolving agents or dissolving aids, isotonic agents, pH adjusting agents, stabilizing agents, propellants, tackifiers, and the like, but not limited to these.

For example, for preparation of pharmaceutical compositions for oral administration such as tablets, capsules, granules, and powders, excipients such as lactose, crystalline cellulose, and starch, lubricants such as magnesium stearate and talc, binders such as hydroxypropylcellulose and polyvinylpyrrolidone, disintegrators such as carboxymethylcellulose calcium and low substituted hydroxypropylmethylcellulose, coating agents such as hydroxypropylmethylcellulose, macrogol, and silicone resin, and the like may be used as required. For preparation of eye drops, isotonic agents such as sodium chloride, potassium chloride, or concentrated glycerin, buffering agents such as sodium phosphate, sodium acetate, boric acid, or monoethanol amine, stabilizing agents such as sodium citrate or edetate sodium, antiseptics such as benzalkonium chloride and a para-oxybenzoic acid, surface active agents such as polysorbate 80 and polyoxyethylene hydrogenated castor oil, pH adjusting agents such as diluted hydrochloric acid or sodium hydroxide and the like may be used as required. A pH of the eye drop is not particularly limited. A range of 4 to 8 is preferred which is acceptable for phthalic preparations.

A dose of the medicament of the present invention is not particularly limited. The dose may be suitably chosen depending on symptoms, age, body weight and the like of a patient, a method for administration, a type of active ingredient and the like. For example, for oral administrations, a dose of 0.01 to 1,000 mg, preferably 0.1 to 100 mg per day may be administered once or several times as divided portions. However, the aforementioned doses are only for examples, and the dose may be appropriately increased or decreased.

### [Examples]

The present invention will be more specifically explained with reference to the examples. However, the scope of the present invention will not be limited to the following examples.

### Example 1 : The control regulation of GDNF by all-trans retinoic acid

Generally, for the system of in vitro astrocyte research, the selection of cells to be used is a matter of importance. Astrocytes that were isolated from human brain are available from the U.S. cell bank or through a research company. However, the cell growth is slow due to the nature of being the primary culture of neural cells that have a well-differentiated character, and therefore, they are not suitable for analysis of gene expression and the like. Therefore, the inventors of the present invention performed experiments by using U373MG cell as a human glioma cell which is GFAP (glial fibrillary acidic protein) positive, that is known as a marker of differentiation into an astrocyte, and also has a stable characteristic as a cell line. U373MG cells are easily available commercially.

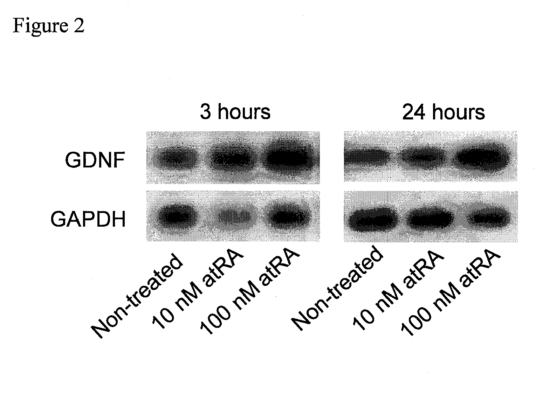

U373MG cells were cultured in a relatively dense state, and were treated with 10 nM or 100 nM of all-trans retinoic acid (atRA). mRNA was extracted from the cells and subjected to quantitative RT-PCR, and then changes in the gene expression were analyzed and quantified by the Southern blot method. Reverse transcription was performed by using a kit manufactured by Invitrogen. The reagents were prepared according to the manufacturer's instructions, and reacted at 42°C for 30 minutes. The product was used for PCR after denaturation of reverse transcriptase at 96°C for 5 minutes. For the PCR reaction, each reagent manufactured by TaKaRa was prepared by mixing according to the manufacturer's instructions, and the following reactions were performed:
1) 94°C, 30 seconds
2) 94°C, 15 seconds
3) 52°C, 15 seconds
4) 72°C, 30 seconds
5) repeat of the reactions from 2) to 4) for 32 times
6) 72°C, 7 minutes
The final reaction product was used for the Southern blot test. The results are shown in Figure 2.

Figure 2 shows the alterations of gene expressions after 3 hours and 24 hours. It is readily understood that GDNF mRNA level expression was increased in relation to the elevated concentration of the all-trans retinoic acid (atRA). It seems that this increase in the expression reached to a plateau level after about 3 hours. For reliable quantification of the alteration of the gene expression, the result of GAPDH (glyceraldehydes-3-phosphate dehydrogenase) is also indicated as an inner control.

One of potential problems in the researches so far of retinoids, including atRA, is that pharmacological concentrations of several hundred times, several thousand, or several tens of thousands times higher concentrations of retinoids than the physiological concentrations have been used for observation of biological reactions induced by the retinoids. If some biological reaction is observed only when a retinoid is exposed at a high concentration for a long period of time, the phenomenon is not reasonably understandable as a physiological reaction. Whilst according to the experimental system provided by the inventors of the present invention, the experiment was performed within a range of a concentration similar to that of atRA concentration in human blood, which is believed to reflect more physiological environment.

### Example 2

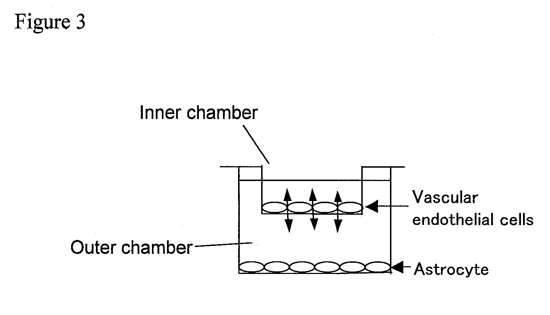

In Example 1, the GDNF expression was increased (dose-dependently) in response to the elevated concentration of atRA. In order to evaluate whether or not the alteration induces physiological reactions, co-cultivation with vascular endothelial was performed. As shown in Figure 3, the double chamber using the transwell was constructed. As can be understood from Figure 3, the U272MG cells (astrocytes) were grown as effector cells in the outer chamber, and treated with atRA and cultured for additional 24 hours. In the inner chamber separately prepared, vascular endothelial cells obtained from the bovine brain were cultured to prepare beforehand for forming a dense single layer cell-sheet.

The outer chamber with U373MG cells and the inner chamber with the vascular endothelial cells were jointed to start the co-culture for evaluation of the barrier function that is a tight-function of the vascular endothelial cells. By culturing the two kinds of cells together, functional interactions of the mutual cells will be revealed. In this experiment, as functional alterations of the vascular endothelial cells by the influence of the astrocyte, the difference in electrical resistance inside and outside of the vascular endothelial cells, and the mobility to the outer chamber of the labeled substance (¹⁴C-labeled) added in the inner chamber were evaluated as indexes as the vascular permeability. The activity of the transferred radioactive substance is indicated as dpm (disintegrations per minutes).

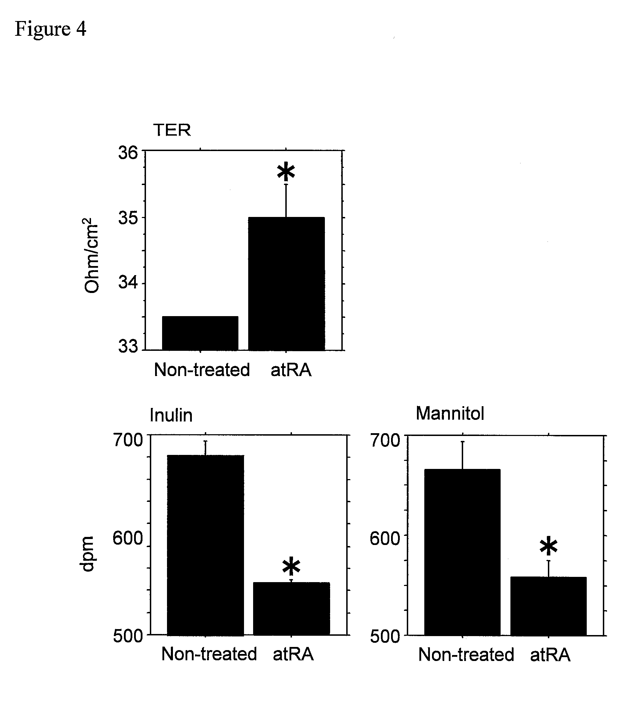

As can be understood from Figure 4, in TER (Transepithelial electrical resistance: TER will become higher when the barrier function is higher) for measuring a barrier function by the electrical resistance, the barrier function of the vascular endothelial cells was enhanced under the co-culture with U373MG cells treated with atRA (*p<0.05). When the permeability of the vascular endothelial cells was evaluated by using the two kinds of substance radioactively labeled, i.e., inulin and mannitol, each permeability of the inulin having a high molecular weight (5kDa) and the mannitol having a low molecular weight (182Da) was significantly suppressed (*p<0.05). From the results of Example 2, it is understood that the alteration of the gene expression of the astrocytes caused by atRA effected in a paracrinic manner so as to sufficiently cause a change in the permeability of the vascular endothelial cells.

### Example 3

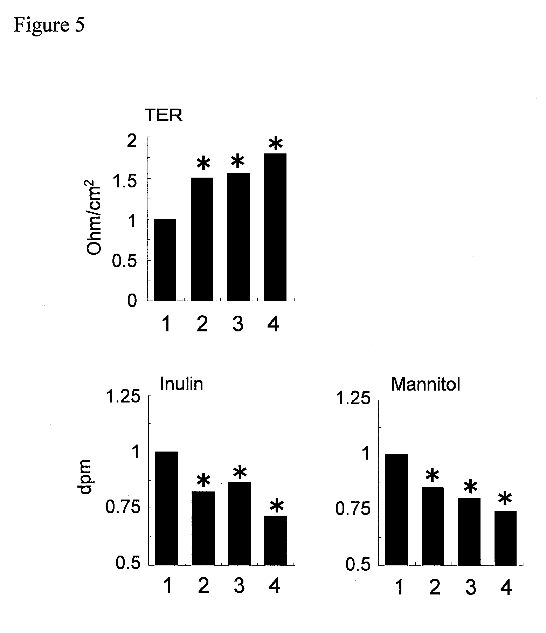

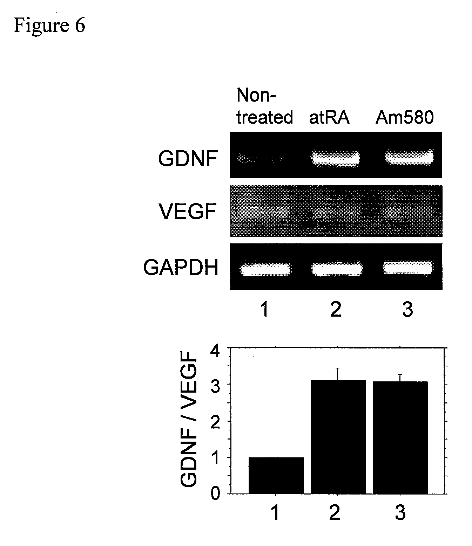

From the results of the co-culture with the vascular endothelial cells in Example 2, the increase in the expression of GDNF induced by atRA was found to be functional to the vascular endothelial cells. From the results of preliminary experiment, it was found that Am580, as well as atRA, induced the expression of GDNF mRNA in a dose-dependent and time-dependent manner (see, Figure 6 below). The vascular endothelial cell used in Example 2 is the primary culture strain obtained from the bovine brain, which has an advantage of capable of reproducing in vitro conditions more similar to those in a living body. However, unlike those called as cell lines, the strain of the primary cultured cell has limited number of division and a slow division rate, and therefore has an aspect of unsuitability for an experimental system that requires repeated examination. Accordingly, MDCK (Madin Darby Canine Kidney) cell as dog-derived kidney tubule cell (easily available commercially) was used, which cell is optimum for the evaluation of the barrier function and has been experienced of being already used in variety of experimental systems. The effect of synthetic retinoids was evaluated with the same parameter as those in Example 2. The results are shown in Figure 5. The experimental groups in Figure 5 are as follows:
1. Non-treated group
2. atRA(RARα agonist)
3. Am80(RARα agonist)
4. Am580(RARα agonist)

In Figure 5, the non-treated group (control) is defined as "1", and relative differences thereto are shown as graphs. In TER, Am580 was more effective than atRA, and much effective than Am80 (*p<0.05). However, when the intercellular permeability was evaluated by using two kinds of labeled substances (inulin and mannitol) having different molecular weights, it was found that Am580 most effectively suppressed the intercellular penetration for the substances with the different molecular weighs (*p<0.05).

### Example 4

In Examples 1 and 2, U373MG cells, a cell line having the characteristic as astrocyte, was used for convenience of the experiment. However, it is known that, in experimental system in vitro, a cell line often does not completely possess original characteristic, and characteristic thereof may be altered also by passages. Therefore, studies were made to evaluate whether or not the enhancement of the expression of GDNF by the retinoid can be observed also in the system using human astrocyte. The astrocyte used in the experiment was a primary cultured cell isolated from the human brain, and was purchased from Cambrex Corporation, USA. This means provides an advantage that observation is achievable under environments closer to those in vivo. The results are shown in Figure 6 below.

As can be understood from Figure 6, atRA and Am580 significantly increases the GDNP expression. Whilst, the expression of VRGF that is a strong vascular hyperpermeability factor was suppressed in the treated groups. The level of suppression of the vascular permeability in the treated groups is represented by a relative amount of the two factors that define permeability, specifically, a value obtained by dividing a value of the expression of GDNF that is the permeability-suppressing factor by a value of the expression of VEGF that is a hyperpermeability factor. In this case, a value of the expression of each of non-treated groups is used as an inner control, and an amount of the expression of the treated groups is calculated from the concentration on the gel and the area. In the atRA- and Am580-treated groups, it was observed that both were superior in the suppression of the vascular permeability about three-fold. Am80 gave similar effect. The experimental groups in Figure 6 are as follows, and the upper figure indicates the photographs of the gel, the lower figure indicates visualized differences of relative amounts obtained by the quantification thereof.
1. non-treated group
2. atRA
3. Am580

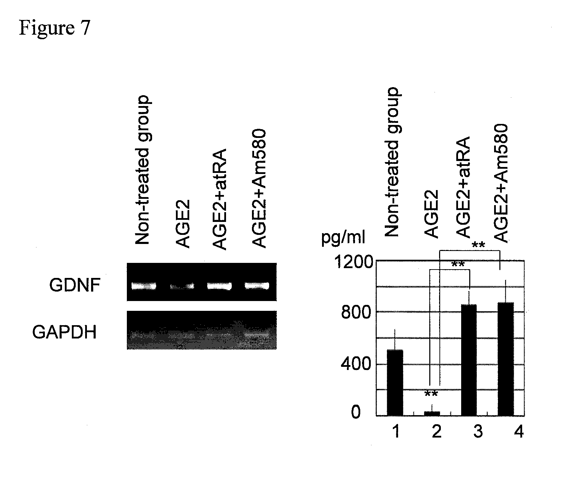

Figure 7 is to confirm that the increase in the expression of GDNF by atRA has biological effects in diabetes. Human astrocyte was treated with AGE-2 alone, or combinations of AGE-2 with atRA or Am580, and alterations in the expression of GDNF was evaluated in the same manner as Figure 6. ARE2 is a glycosylation product observed to be increased in the blood of diabetic patients, and as explained in non-patent document 6, is a substance that suppresses the expression of GNDF. By the experiment, it was found that the effect of AGE-2 to suppress the expression of GDNF was strongly inhibited by atRA and Am580 in mRNA and protein level (**, p<0.001). These results indicate that the increase of the vascular permeability by AGE in the diabetic condition could be suppressed by the retinoids. In Figure 7, the left figure shows the result by RT-PCR method, and the right figure shows the result of EL1SA (enzyme-linked immunosorbent assay) method for quantification of the GDNF protein in the cell culture medium.

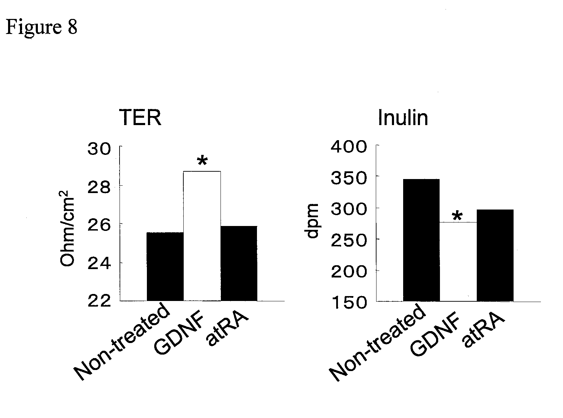

It is known that the vascular endothelial cells also have retinoid receptors, and accordingly, there remains a possibility that atRA contained in the cell culture medium effected directly on the vascular endothelial cells during the co-culture. However, when a recombinant protein of GDNF was directly effected on the vascular endothelial cells in the absence of astrocyte, the increase in TER and the decrease in the permeability were induced (*p<0.05), whilst these changes were not observed when the cells were treated with atRA (Figure 8). From these results, it is understood that the cause that caused the change in the barrier function of the vascular endothelial cells is the astrocyte-derived GDNF of which expression was modified by atRA, and that GDNF acted in a paracrinic manner. In addition, these results support the concept of the biological functional unit that is consisted of glial cells and vascular endothelial cells.

### Example 5: Confirmatory experiment by using diabetic model mice

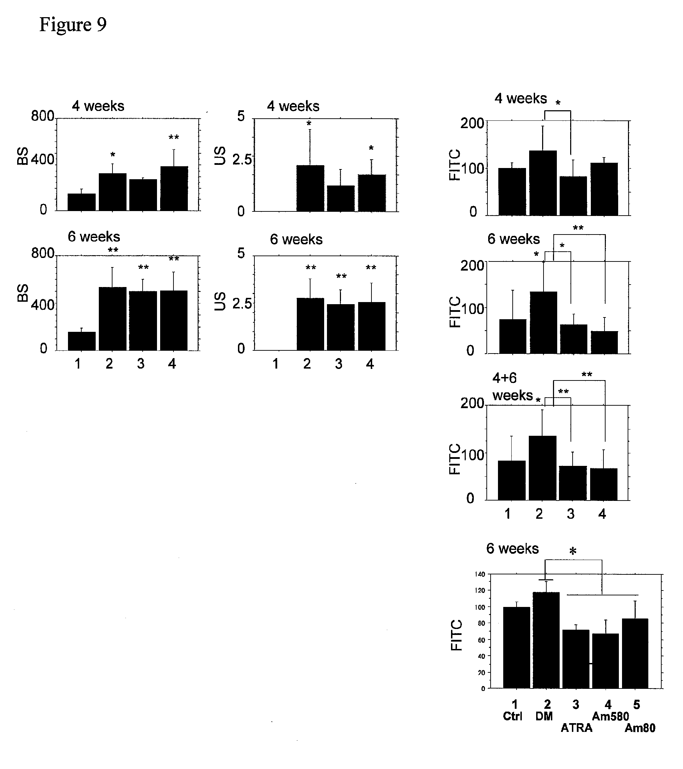

Male 5 to 6 week-old C57/BL6 mice were administered with streptozotocin and diabetes was chemically induce in these model mice. To these diabetic model mice, retinoids were administered to evaluate clinical effectiveness. For reference, mice without the diabetic induction were used as control.
The results are shown in Figure 9. The experimental groups are as follows:
Experimental groups:
   1. Control (group of non-induction of diabetes)
   2. Diabetic group (non-treated group)
   3. Diabetic group (atRA-treated group)
   4. Diabetic group (Am580-treated group)
   5. Diabetic group (Am80-treated group)

The onset of diabetes was confirmed by periodical measurement of glucose in urine, and after all the mice became strongly positive, by measurement of blood glucose. The mice of 4 to 6 week-pathological period with diabetes were used. By rough calculation considering mouse lifetime and so forth, the pathological period of 4 week corresponds to about 5 years of human pathological period, and the 6 weeks corresponds to about 7 to 10 years of pathological period. Mice were divided into 4 groups as mentioned above for the treatment. The treatment were performed by administrating atRA (1 mg/kg), Am580 (3.75 mg/kg), and Am80 (4.5 mg/kg) intraperitoneally every other day for 4 days. Just before the animals were sacrificed, 50 mg/kg of FITC dextran (Mw, 4000Da) was injected via the inferior vena cava under administration of a sufficient amount of systemic anesthesia. As the index of the permeability, the FITC in the eyes was measured, and the value was standardized with the FITC amount contained in cardiac blood to minimize a potential difference between each amount of dose.
As biological parameters, blood sugar (BS) and urine sugar (US) were evaluated. The fact that significantly higher levels of BS and US were observed in the diabetic groups (*p<0.05, **p<0.01) well corresponded to the changes observed in human diabetes. Significantly lower level of US in the atRA-treated group compared to the non-treated group was observed.

In the study of the permeability by the quantification of the amount of FITC in the eyes, the vascular hyperpermeability in the hyperglycemic condition were significantly suppressed in the drug-treated group, i.e., in the early stage diabetic group and middle stage diabetic group as compared to the non-treated group. In middle stage diabetic group, the effectiveness was apparent, and Am580 appears to have higher effectiveness than atRA (*p<0.05, **p<0.01). These pharmacological efficacies are also clearly understood from the data (4+6 weeks) by summing up the early stage and middle stage disease groups. The reason for the above results is considered that Am580 is more stable in vivo in the blood than atRA and a pharmacologically effective blood concentration was maintained for relatively longer period of time. Since RARα receptor per se is already known, one of ordinary skill in the art would readily be able to prepare an RARα agonist that targets to an RARα receptor and it would be obvious that the agonist could be used in the same manner as the compounds of which effectiveness as therapeutic agents for ocular diseases such as diabetic retinopathy was specifically confirmed above according to the present invention.

### [Industrial Applicability]

The medicament of the present invention is useful as a medicament for preventive and/or therapeutic treatment of ocular diseases resulting from vascular hyperpermeability, for example, ocular diseases such as diabetic retinopathy and age-related macular degeneration.

## Claims

1. A medicament for preventive and/or therapeutic treatment of an ocular disease resulting from vascular hyperpermeability, which comprises a retinoid as an active ingredient.

2. The medicament according to claim 1, wherein the ocular disease resulting from vascular hyperpermeability is diabetic retinopathy or age-related macular degeneration.

3. The medicament according to claim 2, which is used for prevention of diabetic retinopathy at early stage diabetes or middle stage diabetes.

4. The medicament according to claim 2, which is used for prevention at presymptomatic stage of diabetic retinopathy.

5. The medicament according to any one of claims 1 to 4, wherein the retinoid is all-trans retinoic acid.

6. The medicament according to any one of claims 1 to 4, wherein the retinoid is a non-natural retinoid.

7. The medicament according to claim 6, wherein the retinoid has a basic skeleton comprising an aromatic ring bound with an aromatic carboxylic acid or tropolone by means of a bridging group.

8. The medicament according to any one of claims 1 to 4, wherein the retinoid is capable of binding to retinoic acid receptor (RAR) subtype α and subtype β.

9. The medicament according to any one of claims 1 to 4, wherein the retinoid is capable of binding to retinoid X receptor X (RXR).

10. The medicament according to any one of claims 1 to 4, wherein the retinoid is Am80 (4-[(5,6,7,8-tetrahydro-5,5,8,8-tetramethyl-2-naphthalenyl)carbamoyl]benzoic acid) or Am580 (4-[(5,6,7,8-tetrahydro-5,5,8,8-tetramethyl-2-naphthalenyl)-carboxyamide]benzoic acid).

11. Use of a retinoid for manufacture of the medicament according to any one of claims 1 to 4.

12. A method for preventive and/or therapeutic treatment of an ocular disease resulting from vascular hyperpermeability, comprising the step of administering an effective amount of the above retinoid to a mammal including a human.
